(19) Europäisches Patentamt / European Patent Office / Office européen des brevets

(11) **EP 2 440 140 B1**

(12) **FASCICULE DE BREVET EUROPEEN**

(45) Date de publication et mention
de la délivrance du brevet:
**24.04.2013 Bulletin 2013/17**

(21) Numéro de dépôt: **10737999.2**

(22) Date de dépôt: **11.06.2010**

(51) Int Cl.:
*A61B 8/08* *(2006.01)*    *G01H 5/00* *(2006.01)*
*G01N 29/46* *(2006.01)*   *G01N 29/06* *(2006.01)*

(86) Numéro de dépôt international:
**PCT/FR2010/051175**

(87) Numéro de publication internationale:
**WO 2010/142927 (16.12.2010 Gazette 2010/50)**

(54) **PROCEDE ET DISPOSITIF ULTRASONORES POUR CARACTERISER UN MILIEU**

METHODE UND ULTRASCHALLGERÄT ZUR CHARACTERISIERUNG EINES MEDIUMS

METHOD AND ULTRASONIC APPARATUS FOR CHARACTERISING A MEDIUM

(84) Etats contractants désignés:
**AL AT BE BG CH CY CZ DE DK EE ES FI FR GB
GR HR HU IE IS IT LI LT LU LV MC MK MT NL NO
PL PT RO SE SI SK SM TR**

(30) Priorité: **11.06.2009 FR 0902856**

(43) Date de publication de la demande:
**18.04.2012 Bulletin 2012/16**

(73) Titulaire: **Centre National de la Recherche
Scientifique
75794 Paris (FR)**

(72) Inventeurs:
• **MINONZIO, Jean-Gabriel
F-75014 Paris (FR)**
• **TALMANT, Maryline
F-27200 Vernon (FR)**
• **LAUGIER, Pascal
F-75017 Paris (FR)**

(74) Mandataire: **Brochard, Pascale et al
Osha Liang
32 avenue de l'Opéra
75002 Paris (FR)**

(56) Documents cités:
WO-A1-03/045251     WO-A1-03/099133
US-A1- 2008 097 211     US-B1- 6 449 566

• PEREIRA W C D A ET AL: "Performance of
ultrasound echo decomposition using singular
spectrum analysis" ULTRASOUND IN MEDICINE
AND BIOLOGY, NEW YORK, NY, US LNKD- DOI:
10.1016/S0301-5629(01)00423-9, vol. 27, no. 9, 1
septembre 2001 (2001-09-01), pages 1231-1238,
XP004308804 ISSN: 0301-5629

**Description**

La présente invention concerne un procédé et un dispositif ultrasonores pour caractériser un milieu.

La présente invention s'applique notamment à la caractérisation de milieux solides constituant des objets et dont il s'agit de connaître la nature, la composition, le degré d'homogénéité, l'état, une dimension etc., en particulier par comparaison avec des résultats établis antérieurement pour des milieux connus.

Il peut s'agir en particulier de détecter de manière non-destructive l'état d'une pièce mécanique. Plus particulièrement, il peut s'agir d'explorer l'état d'un os humain ou animal, en particulier l'état de la couche corticale de l'os, par exemple pour connaître le stade d'évolution d'un os chez l'enfant, l'état d'un os dans le cas de certains handicaps affectant le squelette, ou encore déceler des pathologies osseuses telles que l'ostéoporose et évaluer le cas échéant le degré de gravité de la pathologie détectée.

Les procédés ultrasonores offrent de nombreux avantages par rapport aux procédés radiographiques qui nécessitent un matériel coûteux et un environnement adapté, qui ont des effets secondaires indésirables et dont la fréquence d'application sur un même individu doit être limitée.

On connaît des procédés de caractérisation consistant à appliquer une excitation ultrasonore en un site d'émission et à détecter en plusieurs sites de réception successifs l'arrivée du premier signal généré par cette excitation, puis calculer la vitesse de propagation de ce premier signal. Le procédé peut être mis en oeuvre en appliquant une sonde sur la surface extérieure du corps du patient. Le FR 2 839 877 enseigne un procédé et un dispositif qui éliminent l'influence d'une épaisseur éventuellement variable de tissus mous (peau, muscle) entre la sonde et l'os à explorer. Dans la couche corticale d'un os, la vitesse du premier signal varie en fonction de paramètres tels que l'épaisseur de la couche corticale, la présence d'ostéoporose etc. La seule mesure de la vitesse du premier signal ne permet donc pas toujours de discriminer différents symptômes.

Un autre document partinent est le WO 03/045251 A1.

Or le signal ultrasonore observé aux récepteurs en réponse à une excitation telle qu'une impulsion ou autre excitation multifréquentielle contient plusieurs contributions associées à des ondes qui se propagent à des vitesses différentes, variables en fonction de la fréquence, et avec des niveaux d'énergie différents. Il est connu de détecter les états d'excitation, successifs dans le temps, des multiples récepteurs répartis sur le trajet des signaux, et d'établir avec le résultat de ces détections une image des niveaux d'énergie, en fonction du temps et de l'espace, des signaux ultrasonores transmis le long du milieu d'étude. Cette image spatio-temporelle des niveaux d'énergie est riche d'informations car elle regroupe les conséquences, en termes d'excitation, des différents modes vibratoires suscités par le signal d'excitation dans le milieu d'étude. On sait extraire des signaux reçus par les détecteurs successifs une cartographie des niveaux d'énergie en fonction de la fréquence et de la vitesse de propagation. Cette cartographie prend par exemple la forme d'une représentation, par des niveaux de couleur ou de gris, de l'énergie en chaque point d'un repère fréquence-vitesse de propagation. On y voit apparaître des lignes de forte énergie, appelées « trajectoires », qui reflètent chacune un mode vibratoire et qui forment un ensemble caractéristique du milieu de propagation.

Dans une réalisation visant à l'obtention de cette cartographie, l'image spatio-temporelle peut être traitée en particulier par transformation de Fourier bidimensionnelle numérique pour en extraire un spectre des fréquences temporelles et des fréquences spatiales, et notamment établir une correspondance entre les valeurs des fréquences spatiales et les valeurs des fréquences temporelles. Les signaux reçus, échantillonnés dans le temps et, du fait du nombre fini de récepteurs, dans l'espace, sont mis sous la forme d'une matrice (temps - espace) des niveaux d'énergie dans l'espace et dans le temps. On réalise ensuite une transformation de Fourier numérique à deux dimensions (TF2D) de la matrice des niveaux d'énergie, de façon à passer des variables temps et espace aux variables fréquence et vitesse de propagation. La cartographie précitée correspond à une visualisation de la transformée de Fourier à deux dimensions (TF2D).

Cette technique présente toutefois un certain nombre de limitations et/ou inconvénients. Les modes faiblement énergétiques tendent à être masqués par les modes plus énergétiques. La qualité de l'image obtenue se dégrade dans les milieux bruités. En outre, la technique connue demande une grande quantité d'acquisitions, surtout si le nombre de détecteurs est limité par la forme ou les dimensions de l'objet incorporant le milieu à caractériser. En particulier, une technique pour rendre la méthode connue plus performante consiste à effectuer chaque mesure un grand nombre de fois puis à traiter mathématiquement (TF2D) une matrice formée par les moyennes des mesures effectuées. Ceci élimine en grande partie les erreurs survenant aléatoirement, mais pas certaines sources d'erreurs systématiques.

Le but de la présente invention consiste à remédier à certains au moins des inconvénients précités en proposant un procédé et/ou un dispositif de caractérisation par voie ultrasonore qui soit fondamentalement plus performant que la technique connue.

Suivant l'invention, le procédé ultrasonore de caractérisation d'un milieu, comprend les étapes décrites dans la revendication 1.

On sait que la réponse à une excitation ultrasonore s'analyse comme une addition de composantes vibratoires ayant chacune une amplitude (niveau d'énergie), une fréquence, un positionnement dans le temps (phase), et une vitesse de propagation.

L'excitation (par exemple une impulsion de Dirac) s'analyse elle-même comme formée de composantes vibratoires ayant chacune une fréquence et une amplitude (niveau d'énergie). L'excitation se caractérise aussi par le site d'émission où elle est appliquée, car au sens de l'invention on considère que deux signaux d'excitation sont différents s'ils sont identiquement composés mais appliqués en des sites d'émission différents.

Un deuxième signal d'excitation, différent d'un premier signal d'excitation, génère dans chaque détecteur une réponse différente de celle générée dans ce même détecteur en réponse au premier signal d'excitation.

Cette propriété peut être exploitée de différentes manières. On peut par exemple réaliser plusieurs fois la méthode connue, en changeant le signal d'excitation d'une fois à l'autre, obtenir à chaque fois la cartographie qui correspond au signal d'excitation émis, puis comparer et/ou additionner les cartographies obtenues pour générer une cartographie résultante plus fiable.

L'invention prévoit de préférence d'utiliser plus de deux signaux d'excitation différents, par exemple environ une dizaine, pour améliorer encore les performances de base de l'invention.

Il est préféré selon l'invention que les signaux d'excitation différents diffèrent par la distance entre leur site d'émission respectif et chaque site de réception. Il est également préféré, dans ce cas, que les signaux d'excitation ne présentent pas d'autre différence entre eux, c'est-à-dire que leur spectre de composantes vibratoires élémentaires soit sensiblement le même. On peut par exemple systématiquement utiliser comme signal d'excitation une impulsion de Dirac. On sait que la réponse d'un transducteur ultrasonore à une telle impulsion présente un spectre formé de fréquences situées dans un certain intervalle avec une répartition gaussienne des amplitudes de part et d'autre d'une fréquence centrale.

Comme la réponse à une excitation dans un milieu donné est formée de composantes vibratoires qui se propagent à des vitesses différentes, les composantes vibratoires des deux réponses à deux signaux d'excitation identiquement composés mais appliqués à des distances différentes d'un détecteur donné sont différemment additionnées au passage devant ce détecteur. Les deux signaux captés par chaque détecteur sont donc différents. Là encore, on peut employer plus de deux signaux différents, par exemple une dizaine de signaux appliqués chacun à un site d'émission respectif.

Ainsi, dans le cas général, cette version préférée du procédé utilise $N^E$ émetteurs et $N^R$ récepteurs. De préférence, les émetteurs sont activés successivement, un seul à la fois, et chaque fois qu'un émetteur est activé, on procède à l'acquisition de $N^R$ signaux temporels, un sur chacun des $N^R$ récepteurs. Au total, on obtient $N^E$ X $N^R$ signaux temporels.

En pratique, pour affiner la détection, chaque mesure peut être effectuée un nombre prédéterminé de fois et chaque signal temporel précité, retenu pour la suite du procédé, peut être constitué par la moyenne des signaux obtenus en réponse à ce nombre prédéterminé d'excitations identiques. Dans la suite, pour la clarté de l'exposé, on ne mentionnera plus cette possibilité que chaque signal temporel acquis pour la suite du procédé soit en fait une moyenne de plusieurs signaux effectivement détectés.

On sait que toute fonction temporelle peut s'analyser comme une addition de fonctions sinusoïdales du temps, ayant une amplitude, une fréquence, et une phase (positionnement dans le temps). L'amplitude et la phase peuvent être exprimées comme le module et l'argument, respectivement, d'un même nombre complexe qu'on appellera «amplitude complexe». On rappelle qu'un nombre complexe est composé d'une partie réelle et d'une partie imaginaire. On peut le représenter comme un vecteur dans un repère défini par un axe réel et un axe imaginaire, qui se croisent perpendiculairement au point d'origine du vecteur. Le module est la longueur du vecteur, l'argument est l'angle qu'il forme avec l'axe réel.

La transformation de Fourier, réalisable numériquement par ordinateur, permet de connaître l'amplitude complexe associée à chaque fonction sinusoïdale composant une fonction. On appelle transformation de Fourier temporelle cette transformation appliquée à une fonction du temps pour obtenir une somme de composantes vibratoires. Le pas des fréquences (intervalle entre deux fréquences successives) des fonctions sinusoïdales qui apparaîtront dans la transformée de Fourier temporelle est choisi à volonté, en général dans le cadre d'un compromis entre la finesse des résultats souhaitée, la durée du calcul, et le cas échéant les limites de capacité des moyens informatiques utilisés.

Dans une version préférée de l'invention, on détermine, en particulier par la transformation de Fourier temporelle, le spectre des composantes vibratoires de chaque signal ultrasonore reçu, autrement dit chacun des $N^E$ X $N^R$ signaux temporels mentionnés plus haut. La série de fréquences utilisée pour toutes les transformations de Fourier est la même. Ainsi pour chaque fréquence de la série, on obtient une amplitude complexe associée à chacun des $N^E$ X $N^R$ signaux temporels.

Il en résulte, pour chaque fréquence, $N^E$ X $N^R$ amplitudes complexes qui peuvent être mises sous forme d'une matrice de dimension $N^E$ X $N^R$.

Par rapport à la technique antérieure dans laquelle on passait directement des signaux de mesure à la cartographie au moyen d'une transformée de Fourier bidimensionnelle, l'invention propose donc, à ce stade, de passer par une transformée de Fourier unidimensionnelle, à savoir simplement temporelle, et non pas spatio-temporelle.

On a constaté que l'énergie qui se propage dans un milieu a un comportement linéaire en fonction de la fréquence. Ceci signifie que l'énergie associée à une composante vibratoire dans le signal d'excitation se retrouve dans les composantes vibratoires de même fréquence de la réponse à cette excitation. Cette particularité permet d'appliquer le calcul matriciel.

Si on connaissait une vitesse de phase, on serait capable d'exciter l'ensemble des émetteurs à la fréquence correspondante et de manière coordonnée, notamment en termes de positionnement temporel (phase), pour que chaque émetteur à partir du deuxième renforce le signal passant devant lui en provenance du ou des émetteur(s) situé(s) en amont. On pourrait ainsi d'une part amplifier l'excitation et d'autre part mieux observer la réponse du milieu à cette excitation, notamment en termes de niveau énergétique.

Il a été trouvé que le calcul matriciel permettait de simuler cette recherche et ainsi de repérer avec précision les vitesses de déplacement de l'énergie à chaque fréquence dans le milieu à caractériser.

Le calcul matriciel enseigne une opération appelée « décomposition en valeurs singulières » (SVD) applicable à toute matrice. Suivant cette technique mathématique, la matrice de dimension M x N est décomposée en un certain nombre de triplets comprenant chacun une valeur singulière et deux vecteurs singuliers associés. L'un des vecteurs est de dimension M, l'autre vecteur est de dimension N. Le nombre de triplets est égal à la plus petite des deux dimensions M et N de la matrice.

En appliquant cette décomposition en valeurs singulières à chacune des matrices précitées, associées chacune à une fréquence, on obtient pour chaque matrice (chaque fréquence) $N^E$ triplets comprenant un vecteur émission à $N^E$ coordonnées complexes, un vecteur réception à $N^R$ coordonnées complexes et une valeur singulière qui est un nombre réel positif.

Pour une fréquence donnée, les $N^E$ vecteurs émission constituent une base de vecteurs orthogonaux de l'espace mathématique des excitations.

La valeur singulière est indicative du niveau énergétique des modes associés à cette fréquence.

Pour chaque fréquence, les $N^E$ vecteurs singuliers de réception constituent une base de vecteurs orthogonaux dans l'espace mathématique des réponses.

Ensuite, on recherche à quelle onde plane sont associés les vecteurs singuliers de réception. Un vecteur représentant une onde plane à chaque point de réception est constitué. On calcule sur la base des vecteurs singuliers de réception les coordonnées du vecteur à fréquence donnée et à vitesse de phase fixée. Ce faisant, pour chaque fréquence, on explore toutes les ondes planes en faisant varier la vitesse de phase.

On associe à chaque valeur du couple (fréquence, vitesse de phase), la somme des coordonnées au carré ainsi calculé. On constitue ainsi une image en niveaux de gris, les valeurs du niveau étant comprises entre 0 et 1. L'image ainsi obtenue fait apparaître les courbes de vitesse de phase des modes qui se sont propagés le long du réseau de récepteurs.

Dans le repère fréquence-vitesse de propagation, toutes les trajectoires n'ont pas un même niveau énergétique, et le niveau peut même varier fortement le long d'une trajectoire. Une visualisation des niveaux d'énergie réels tels qu'on les obtient directement par la technique antérieure de la TF2D manque en pratique de clarté, car certaines trajectoires peu énergétiques pourront ne pas apparaître et même certaines trajectoires moyennement énergétiques pourront être masquées par l'environnement de trajectoires fortement énergétiques. Pour cette raison, il est avantageux de normer la représentation des niveaux d'énergie, par exemple en donnant la valeur 1 au niveau maximum pour chaque fréquence.

La solution proposée norme automatiquement les niveaux représentés sur la cartographie car les niveaux d'énergie sont exprimés dans les valeurs singulières et celles-ci, dans la solution préférée selon l'invention, sont à un certain stade éliminées du calcul des niveaux représentés par la cartographie.

Suivant une particularité importante de l'invention, on ne conserve pour la cartographie que les réponses fréquentielles ayant les plus forts niveaux d'énergie. Dans le mode de réalisation comprenant la décomposition en valeurs singulières des matrices de réponses fréquentielles, le filtrage des réponses fréquentielles pour ne conserver que les plus énergétiques peut s'effectuer très simplement en ne conservant que les vecteurs singuliers de réception associés à une valeur singulière dépassant un certain seuil. On peut par exemple, pour chaque fréquence, ne conserver qu'un nombre prédéterminé de vecteurs singuliers de réception, associés aux valeurs singulières les plus élevées qui ont été trouvées pour cette fréquence. En variante, on peut pour chaque fréquence ne conserver que les vecteurs singuliers de réception qui sont associés à des valeurs singulières dépassant un certain seuil prédéterminé qui peut être le même pour toutes les fréquences, ou varier selon les fréquences ou les plages de fréquences.

Ce filtrage permet d'éliminer de l'analyse les modes faiblement énergétiques car on considère qu'ils correspondent à du bruit.

Le niveau représenté en chaque point de l'image exprime le degré de contribution d'une onde plane donnée dans la base des vecteurs singuliers de réception. Plus il est élevé et plus le mode de vibration contribue à la réponse vibratoire.

On peut encore améliorer la qualité des résultats en ne conservant dans l'image résultante que les valeurs supérieures à un certain seuil prédéterminé, par exemple 0,6 ou 0,7. On peut choisir de ne représenter que les modes à plus forte contribution en appliquant ce seuil. Tous les points qui ne sont pas retenus sont par exemple visualisés comme si leur niveau était nul.

Les zones de forte énergie ont un peu la forme d'une chaîne de montagne ayant une crête qui correspond à une trajectoire telle que définie plus haut. On peut traiter les résultats pour ne faire apparaître que les trajectoires.

Suivant un second aspect de l'invention, le dispositif pour la mise en oeuvre du procédé précité, comprenant une sonde équipée de moyens d'émission ultrasonore et d'une rangée de récepteurs ultrasonores, des moyens pour activer les moyens d'émission et recevoir les signaux provenant des récepteurs dans une fenêtre de temps après activation des moyens d'émission, et des moyens de traitement pour extraire des signaux reçus une cartographie des niveaux d'énergie dans un repère fréquence-vitesse de propagation, est caractérisé en ce que les moyens d'émission comprennent plusieurs émetteurs et les moyens de traitement établissent la cartographie en combinant mathématiquement les signaux reçus en réponse à différents états d'activation des émetteurs.

Comme on l'a exposé précédemment, les différents états d'activation des émetteurs peuvent par exemple consister en ce que, à chaque fois, un seul émetteur est activé et les autres sont au repos, l'émetteur activé n'étant pas le même d'une fois à l'autre.

Les moyens de traitement réalisent :

- la transformée de Fourier temporelle de chaque signal reçu par chaque récepteur en réponse à chaque état d'activation, de façon à obtenir pour chaque fréquence une matrice $N^E \times N^R$ des $N^E$ réponses acquises par les $N^R$ détecteurs en réponse aux $N^E$ états d'activation,

- la décomposition en valeurs singulières de ces matrices pour tirer de chacune d'elles au moins un vecteur singulier formant partie constitutive d'une base de l'espace de réception,

- le calcul des coordonnées d'un vecteur représentant une onde plane donnée dans la base des vecteurs singuliers de réception.

Ceci sera à l'origine de l'image fournie comme expliqué plus haut.

D'autres particularités et avantages de l'invention ressortiront encore de la description ci-après, relative à deux exemples de réalisation non limitatifs.

Aux dessins annexés :

-- la figure 1 est une vue schématique d'un dispositif selon l'invention ;

-- la figure 2 montre schématiquement la barrette d'émetteurs et de détecteurs ou récepteurs de la sonde de la figure 1 appliquée sur une plaque de cuivre de 2 mm d'épaisseur ;

-- la figure 3 représente les signaux obtenus sur chacun des récepteurs en réponse à un signal d'excitation appliqué par l'émetteur n° i dans le mode de mise en oeuvre illustré à la figure 2 ;

-- la figure 4 représente les modules des valeurs singulières en fonction de la fréquence ;

-- la figure 5 représente un graphe d'utilisation de la base des vecteurs singuliers de réception ;

-- la figure 6 représente par des niveaux de gris dans un repère fréquence-vitesse de propagation une cartographie du module au carré des composantes des ondes planes dans la base des vecteurs singuliers de réception, après élimination des vecteurs de la base associés à des valeurs singulières inférieures à un seuil fixé ;

-- la figure 7 représente la cartographie de la figure 6 après prise en compte uniquement des ondes planes telles que le vecteur associé a une norme au carré supérieure à 0,7 dans la base des vecteurs singuliers de réception ;

-- la figure 8 représente les trajectoires tirées de la cartographie de la figure 7 ;

-- la figure 9 est un graphe comparatif des résultats obtenus avec la technologie antérieure de la transformée de Fourier bidirectionnelle (TF2D) et avec l'invention ;

-- la figure 10 représente partiellement et schématiquement l'application de la sonde selon l'invention sur le bras d'un patient ;

-- la figure 11 est une vue analogue à la figure 5 mais dans le cas de l'application de la sonde sur un radius in vitro ;

-- la figure 12 représente la cartographie correspondante dans laquelle on a représenté les lignes de niveau du niveau de gris ;

-- la figure 13 représente les trajectoires obtenues par utilisation de la technique selon invention ;

-- la figure 14 représente les trajectoires obtenues par utilisation de la technique antérieure par la TF2D ;

-- la figure 15 représente les trajectoires théoriques correspondantes ;

-- la figure 16 est une vue analogue à la figure 11 mais lorsque l'invention est appliquée à une plaque de 2 mm d'épaisseur d'un matériau « imitant » l'os, les valeurs singulières étant indiquées en décibels ;

-- la figure 17 représente la cartographie correspondante ; et

-- la figure 18 représente les trajectoires obtenues et les trajectoires théoriques de la plaque de matériau imitant l'os.

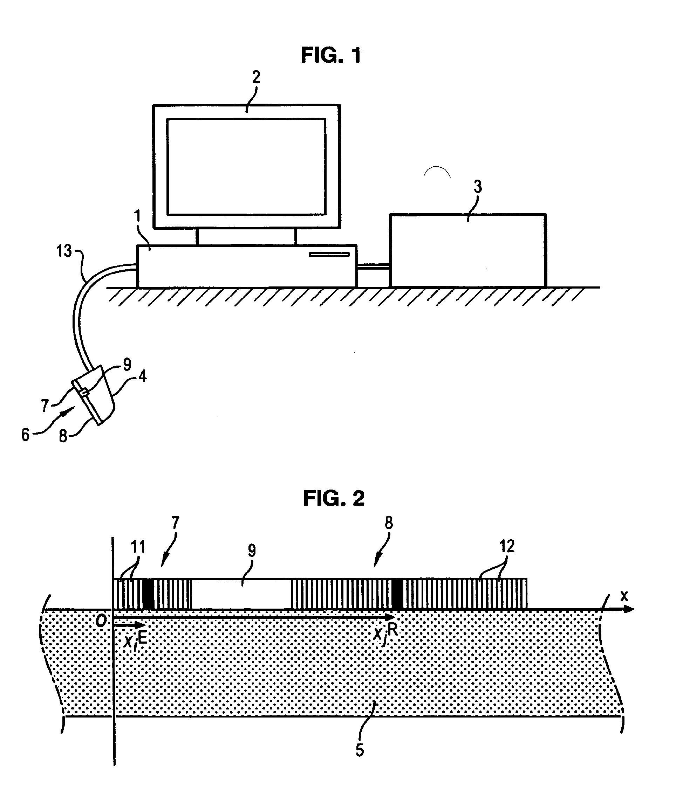

Dans l'exemple représenté aux figures 1 et 2 le dispositif selon invention comprend une unité de traitement 1 qui est reliée à un écran de visualisation 2 et à une imprimante 3, ainsi qu'à une sonde 4.

La sonde 4 comporte une face active 6 qui est en général de forme plane et qui est destinée à être appliquée directement ou indirectement sur le milieu 5 à caractériser. La face active 6 présente principalement une partie émission 7 et une partie réception 8 séparées par une barrière 9 empêchant la transmission ultrasonore directe entre la partie émission 7 et la partie réception 8.

Comme illustré à la figure 2, la partie émission 7 comprend un certain nombre $N^E$ d'émetteurs 11 et la partie réception 8 comprend un certain nombre $N^R$ de récepteurs 12. Pour ne pas charger la figure 2, on n'a référencé qu'un petit nombre des émetteurs 11 et des récepteurs 12. Les émetteurs 11 sont alignés et les récepteurs 12 sont alignés sur la même ligne que les émetteurs 11. Le pas des émetteurs et des récepteurs est par exemple de 0,8 mm. Dans l'exemple, ce pas est le même pour les émetteurs et les récepteurs. Mais il peut être avantageux de choisir des pas différents, en particulier qui ne soient pas multiples l'un de l'autre ni multiples d'un diviseur commun.

L'unité de traitement 1 comprend de manière connue en soi des moyens pour déclencher simultanément l'émission d'une excitation ultrasonore par la partie émission 7 de la sonde, un comptage du temps, et un enregistrement des signaux reçus sur chacun des récepteurs 12 pendant une fenêtre de temps prédéterminée à partir du déclenchement de l'émission.

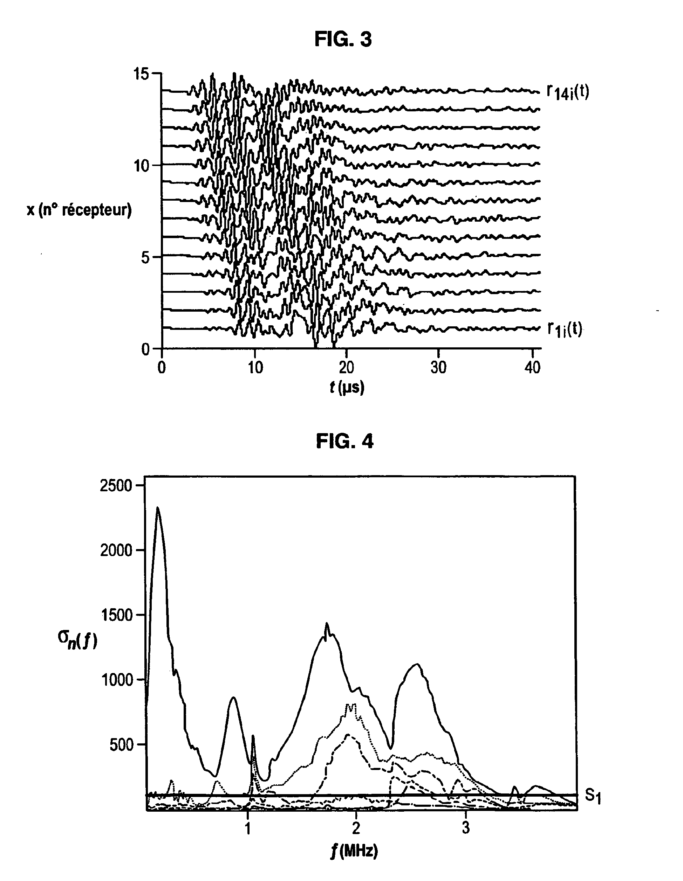

La figure 3 représente la visualisation graphique d'un tel enregistrement pendant une fenêtre de temps de 40 μs, représentée en abscisses, et pour une sonde comprenant quatorze récepteurs 12 dont les numéros sont indiqués en ordonnées. Pour chaque récepteur, le niveau zéro du signal correspond au point de départ du signal associé à ce récepteur sur l'axe vertical des ordonnées.

Chaque émetteur 11 est un transducteur ultrasonore qui transforme un signal électrique reçu de l'unité de traitement 1 en un signal ultrasonore transmis au milieu 5 contre lequel la sonde est appliquée. Chaque récepteur 12 est un transducteur ultrasonore qui transforme un signal ultrasonore reçu du milieu 5 contre lequel la sonde est appliquée en un signal électrique envoyé à l'unité de traitement 1. L'unité de traitement 1 contient des moyens pour traiter les signaux provenant de la partie réception 8 de la sonde 4 et en extraire les variations de vitesse de phase en fonction de la fréquence des ondes ultrasonores qui se sont propagées le long des récepteurs, comme on l'exposera plus en détail plus loin.

Suivant l'invention, l'unité de traitement 1 est capable de générer plusieurs états d'activation de la partie émission 7 de la sonde 4 et d'enregistrer les signaux reçus par l'ensemble des récepteurs 12 de la sonde 4 pour chacun des états d'activation. L'intervalle de temps entre la génération de deux états successifs est au moins aussi grand que la fenêtre de temps (40 μs dans l'exemple de la figure 3) pendant laquelle l'unité de traitement 1 enregistre les signaux reçus.

Dans l'exemple qui va être décrit, chaque état d'activation consiste à activer un seul des émetteurs 11 en laissant les autres au repos. Les états d'activation se distinguent les uns des autres par le numéro de l'émetteur qui est activé. Tous les émetteurs, lorsqu'ils sont activés, émettent un signal d'excitation identique qui est typiquement une réponse à une impulsion de Dirac. Dans cet exemple, le nombre d'états d'activation est donc égal au nombre $N^E$ des émetteurs, mais cela n'est pas limitatif.

Dans la représentation de la figure 2, l'axe des x est parallèle à la direction d'alignement des deux émetteurs 11 et des récepteurs 12, l'indice « i » représente le numéro de l'émetteur 11 considéré, ayant une position $x_i^E$ le long de l'axe des x, et l'indice « j » représente le numéro du récepteur 12 considéré, ayant une position $x_j^R$ le long de l'axe des x.

Ainsi, pour chaque état d'activation, l'unité de traitement 1 enregistre $N^R$ réponses spatio-temporelles formant un ensemble semblable à celui représenté à la figure 3. Une fois que l'unité de traitement 1 a généré les $N^E$ états d'activation correspondant chacun à l'activation de l'un respectif des émetteurs 11, l'unité de traitement 1 a acquis $N^E$ ensembles de $N^R$ réponses spatio-temporelles, donc au total $N^R \times N^E$ réponses spatio-temporelles. On appelle dans la suite $r_{ji}(t)$ la réponse spatio-temporelle acquise par le détecteur n° j en réponse à une activation de l'émetteur n° i. On voit ainsi à la figure 3 les 14 réponses spatio-temporelles $r_{1i}(t)$ à $r_{14i}(t)$ consécutives à l'activation de l'émetteur n° i.

On va maintenant décrire le traitement des réponses spatio-temporelles par l'unité de traitement 1.

Par transformation de Fourier temporelle, chaque signal temporel $r_{ji}(t)$ reçu par un récepteur « j » en réponse à un état d'excitation, dans l'exemple l'excitation d'un émetteur « i », est une addition de composantes vibratoires ayant chacune une fréquence et une amplitude complexe que l'on peut calculer pour chaque fréquence par la formule suivante :

$$R_{ji}(f) = \int_{-\infty}^{+\infty} r_{ji}(t)\, e^{-i2\pi f t}\, dt$$

On obtient ainsi, pour chaque fréquence (f), $N^R \times N^E$ amplitudes complexes que l'on peut écrire sous la forme de la matrice R(f) suivante ayant $N^R$ lignes et $N^E$ colonnes :

$$\begin{pmatrix} R_{11} & R_{12} & \ldots & R_{1N^E} \\ R_{21} & R_{22} & \ldots & R_{2N^E} \\ & \ldots & & \\ R_{N^R1} & R_{N^R2} & \ldots & R_{N^RN^E} \end{pmatrix}$$

Suivant les règles de la décomposition en valeurs singulières, chacune des matrices R(f) de réponse fréquentielle peut s'écrire sous la forme suivante, correspondant à une somme de $N^E$ termes dont chacun est une matrice :

10

$$R(f) = \sum_{n=1}^{N^E} U_n(f) \cdot \sigma_n(f) \cdot {}^t V_n(f)^*$$

Chaque terme de la sommation est formé d'un vecteur (matrice colonne) $U_n(f)$ multiplié par un nombre réel positif $\sigma_n(f)$ multiplié par une matrice ligne notée comme la transposée de la conjuguée d'un autre vecteur $V_n(f)$.

Dans cette expression, $U_n(f)$ est un vecteur dit « singulier » à $N^R$ coordonnées complexes qu'on appellera « vecteur réception », $V_n(f)$ est un vecteur dit « singulier » à $N^E$ coordonnées complexes qu'on appellera « vecteur émission », et $\sigma_n(f)$ est une valeur singulière. On voit qu'il y a pour chaque fréquence f un nombre $N^E$ (le nombre d'états d'activation qui est égal au nombre d'émetteurs dans l'exemple) de triplets formés chacun d'un vecteur singulier émission, d'un vecteur singulier réception, et d'une valeur singulière.

Il a été trouvé selon l'invention que les vecteurs singuliers émission sont chacun descriptifs d'une excitation combinée des différents émetteurs qui stimule un mode de la réponse, chaque valeur singulière représente le niveau énergétique du mode associé au vecteur singulier émission correspondant, et chaque vecteur singulier réception peut être considéré comme l'un des vecteurs unitaires d'une base de l'espace des signaux de réception. Les vecteurs unitaires de chaque base associée à une fréquence sont orthogonaux entre eux.

On procède ensuite à une première filtration consistant à ne conserver que les vecteurs singuliers qui sont associés aux valeurs singulières les plus élevées, correspondant, comme on l'a vu, aux réponses les plus énergétiques.

La figure 4 représente les valeurs singulières $\sigma_n(f)$ en fonction de la fréquence. On décide arbitrairement un seuil $S_1$. On considère que les valeurs singulières inférieures au seuil $S_1$ sont associées à des phénomènes de bruit et on élimine les vecteurs réception correspondants pour la suite du traitement mathématique.

Le nombre de valeurs singulières d'une matrice est égal à la plus petite de ses deux dimensions. Dans l'exemple, il y a $N^E=8$ émetteurs et $N^R=14$ récepteurs, et la plus petite des dimensions de la matrice est donc $N^E = 8$. Il y a donc pour chaque fréquence huit valeurs singulières. Après la filtration, on obtient, pour chaque fréquence, un espace de réception ayant un nombre $N^{EK}$ de dimensions qui n'est pas toujours le même, qui est inférieur ou éventuellement égal au nombre $N^E$ d'états d'activation, et qui est inférieur au nombre $N^R$ de récepteurs.

On pourrait procéder à ce premier filtrage d'une manière un peu différente, par exemple en faisant varier le seuil en fonction de la fréquence, ou en ne retenant pour chaque fréquence qu'un nombre prédéterminé de valeurs singulières, ce nombre étant le même pour toutes les fréquences, ou variable en fonction de la fréquence ou par plages de fréquences. Quel que soit le mode de filtrage utilisé, on obtient pour chaque fréquence une base de $N^{EK}$ vecteurs singuliers réception pour les ondes se propageant à cette fréquence le long du réseau des récepteurs.

Dès lors, il est possible d'exprimer une onde plane quelconque (associée à un couple fréquence f, vitesse de phase c) dans la base associée à cette fréquence. Cette onde s'exprime par un vecteur $e^{test}(f,c)$ à $N^R$ coordonnées dont la coordonnée de rang j est donnée par l'équation :

$$e^{test}(f,c,j) = \frac{1}{\sqrt{N^R}} \exp\left[i\frac{2\pi f}{c}x_j\right]$$

La division par la racine de $N^R$ est destinée à faire en sorte que la somme des carrés des $N^R$ composantes, c'est-à-dire le module du vecteur, soit égale à 1.

Dans la base des vecteurs singuliers de réception, le vecteur test $e^{test}(f,c)$ s'exprime de la façon suivante :

$$e^{test}(f,c) = \sum_{n=1}^{N^{EK}} \left\langle e^{test}(f,c) \middle| U_n(f) \right\rangle U_n$$

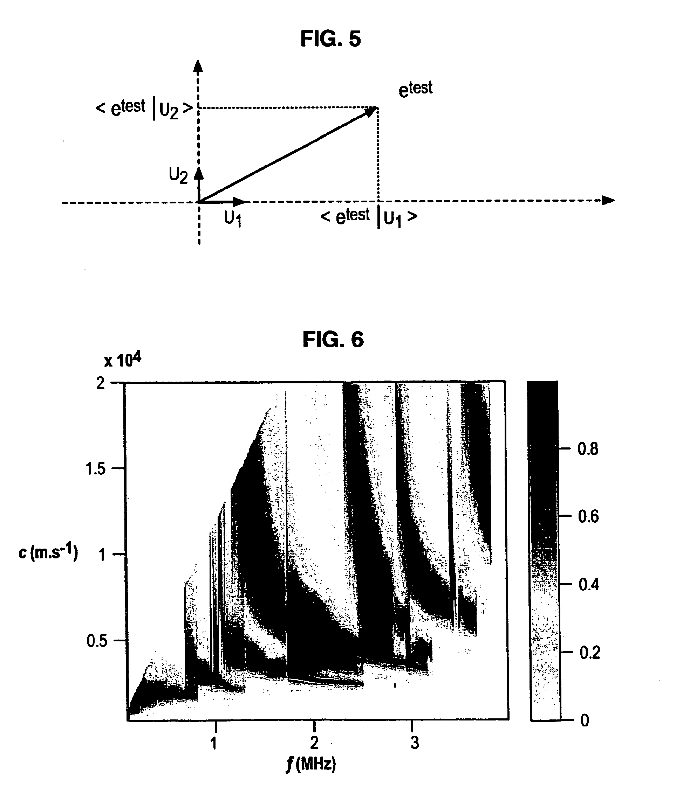

La notation $\langle\cdots|\cdots\rangle$ indique un produit scalaire. La figure 5 illustre cette opération consistant à projeter le vecteur test sur les deux axes d'une base à deux dimensions ($N^{EK} = 2$).

Il est intéressant de noter que

$$\left\langle e^{test}(f,c) \middle| U_n(f) \right\rangle = \tilde{U}(f,c)$$

expression dans laquelle $\tilde{U}(f,c)$ est la transformée de Fourier spatiale du vecteur singulier $U_n$.

Dans cet exemple, chaque point (fréquence fixée, vitesse de phase fixée) de l'image construite est défini par :

$$im(f,c) = \sum_{n=1}^{N^{EK}} \left| \left\langle e^{test}(f,c) \middle| U_n \right\rangle \right|^2$$

C'est le carré du module de la projection du vecteur test dans la base des $N^{EK}$ vecteurs singuliers de réception conservés. Ceci est en partie arbitraire, on aurait pu aussi, par exemple, prendre comme valeur représentative le module lui-même, donc la racine carrée de la valeur correspondant à la formule ci-dessus. Le module du vecteur test étant égal à 1, le module de sa projection dans la base des vecteurs singuliers de réception conservés est toujours inférieur ou égal à 1. Le carré de ce module est donc, également, toujours inférieur ou égal à 1.

La figure 6 représente l'image ainsi obtenue, qui peut être visualisée sur l'écran 2 de la figure 1, ou faire l'objet d'une impression graphique par l'imprimante 3 de la figure 1. Une échelle à droite de la figure explicite la valeur associée à chaque niveau de gris. Les niveaux de gris sont compris entre 0 et 1 car l'image représente la norme au carré d'un vecteur représentant une onde plane, norme qui ne peut pas être supérieure à 1 par construction selon l'exposé ci-dessus. Il y a en bas de la figure et à gauche de la figure deux zones triangulaires entièrement blanches correspondant à des points qui n'ont pas été calculés car ils sont peu significatifs.

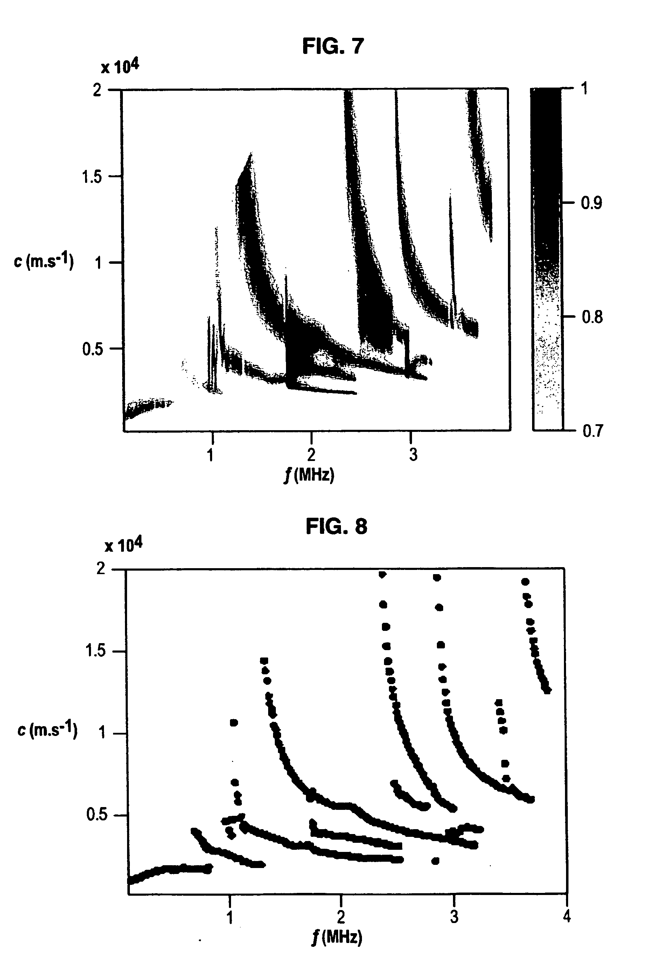

Comme le montre la figure 7, on peut aussi, à ce stade, effectuer un second filtrage, en appliquant, cette fois-ci sur l'image résultante, un second seuil $S_2$ choisi égal à 0,7 dans cet exemple. On ne conserve alors, parmi les ondes planes qui participent aux vecteurs singuliers de réception, que celles qui donnent la plus forte contribution (via les valeurs les plus élevées de la norme au carré du vecteur test).

On voit à la figure 7 que les points correspondant à un niveau de gris supérieur au second seuil $S_2$ ont une forme générale de chaînes de montagnes ayant une crête. Dans la représentation de la figure 8, on n'a conservé que les crêtes et on obtient ainsi une représentation des trajectoires telles qu'on les a définies plus haut.

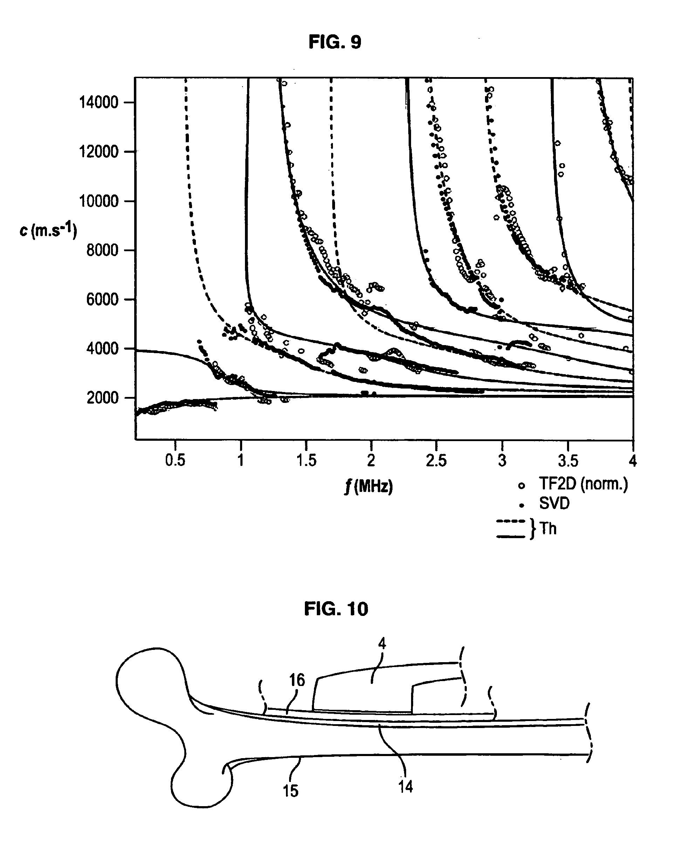

Dans la représentation de la figure 9, les trajectoires théoriques connues pour une plaque de cuivre de 2 mm d'épaisseur apparaissent en trait plein et en pointillé. Les trajectoires obtenues avec la méthode antérieure par la TF2D sont définies par des petits cercles figurant chacun un point. Les trajectoires obtenues avec la méthode selon l'invention, passant par la décomposition en valeurs singulières (SVD) sont définies par des points noirs. Il apparaît d'une part que l'invention fait apparaître des trajectoires plus proches de la théorie, donc probablement plus exactes, et d'autre part que l'invention révèle un mode que la méthode antérieure n'a pas du tout permis de détecter, dans la zone comprise entre 2,5 et 3 MHz.

La figure 10 montre l'application de la sonde 4 sur un patient, au-dessus de la couche corticale 14 d'un os 16 qui peut être par exemple un fémur, un radius, ou etc. Une certaine épaisseur de tissus mous 16 est interposée entre la sonde 4 et la surface extérieure de la couche corticale 14.

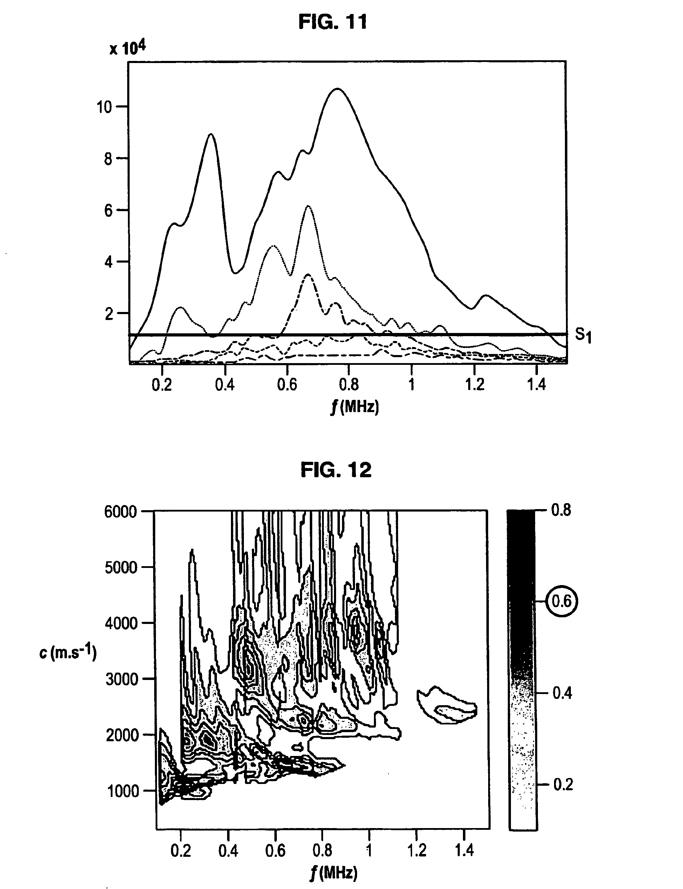

La figure 11 représente les valeurs singulières obtenues en fonction de la fréquence avec une sonde à 5 émetteurs appliqués directement sur un radius dans le cadre d'une expérience in vitro. Comme il y a cinq émetteurs, il y a cinq valeurs singulières pour chaque fréquence. La figure montre également un exemple de seuil $S_1$ constant pour toutes les fréquences. Les vecteurs singuliers qui correspondent à des valeurs singulières situées en dessous de ce seuil ne sont pas retenus pour la suite du traitement mathématique.

La figure 12 représente les images obtenues après un traitement mathématique conforme à celui expliqué plus haut. Sur l'échelle de droite, le niveau de gris égal à 0,6 est entouré pour indiquer qu'il peut être choisi comme second seuil $S_2$ en dessous duquel un point est représenté comme ayant un niveau de gris égal à zéro, sensiblement comme exposé précédemment en référence à la figure 7.

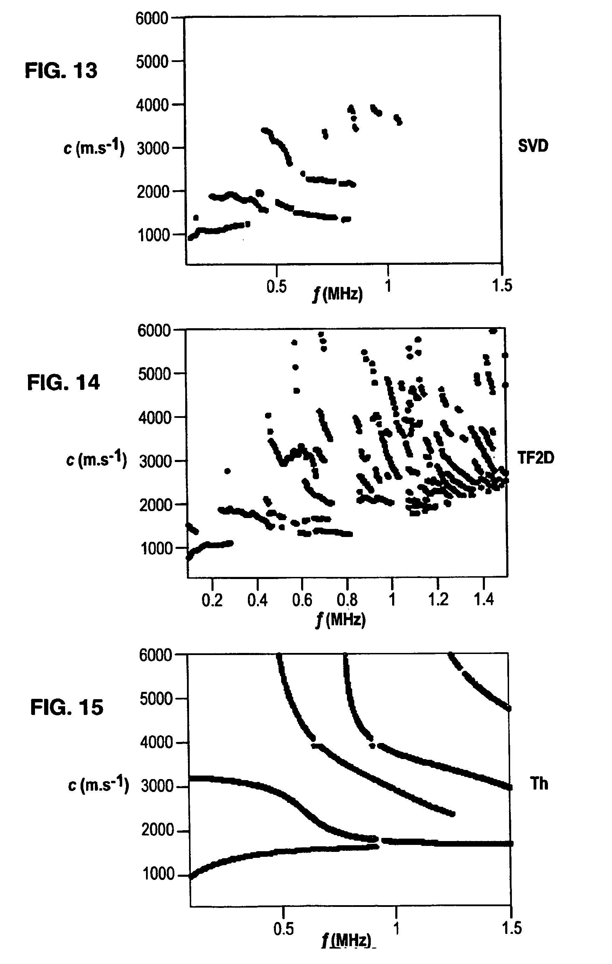

La figure 13 représente les trajectoires correspondant à la visualisation de la figure 12. La figure 14 représente les trajectoires obtenues dans des conditions expérimentales comparables en utilisant la méthode antérieure de la TF2D. Les trajectoires des figures 13 et 14 sont à comparer aux trajectoires théoriques de la figure 15. Alors que le résultat obtenu avec l'invention (figure 13) permet de reconnaître les modes théoriques, le résultat obtenu avec l'art antérieur (figure 14) est difficilement exploitable car encombré de points parasites.

Ainsi, le procédé selon l'invention démontre son aptitude à fournir un résultat plus précis, dont le bruit est largement éliminé.

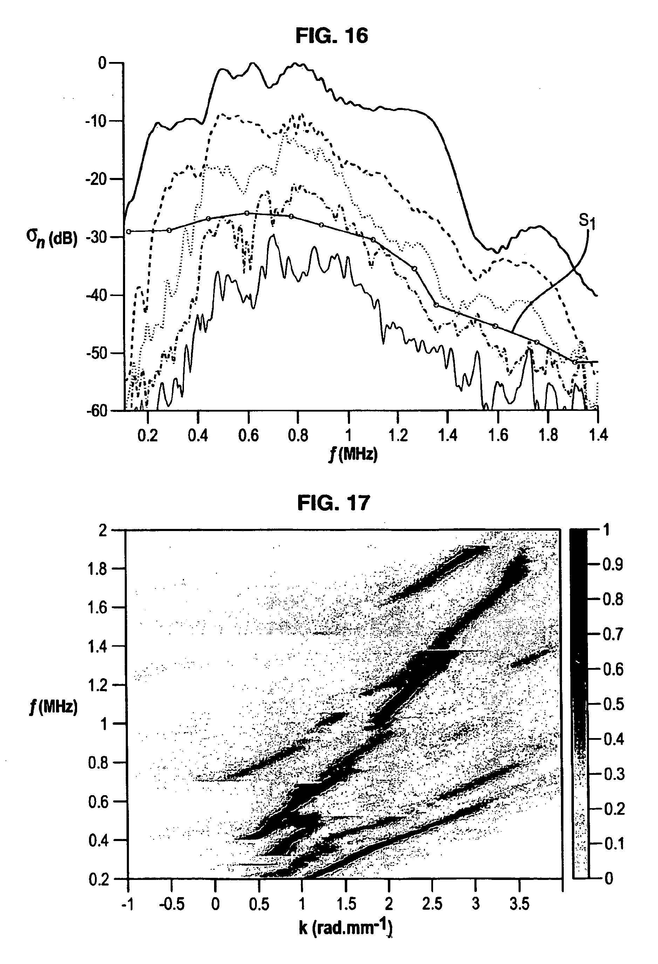

Les figures 16 à 18 sont relatives à des essais ayant consisté à mettre en oeuvre l'invention sur une plaque de 2 mm d'un matériau fabriqué par Sawbones, une division de Pacific Research Laboratoires, Inc., sise à Vashon, Etat de Washington, Etats-Unis. Ce matériau synthétique a été développé dans le but de présenter des caractéristiques physico-mécaniques aussi proches que possible de celles de l'os.

La figure 16 représente les valeurs singulières en fonction de la fréquence temporelle. Les valeurs singulières sont exprimées en décibels d'affaiblissement par rapport à la valeur 1 (correspondant à 0 décibel d'affaiblissement). Cette représentation en décibels a pour but de visualiser les faibles valeurs plus clairement qu'à la figure 11 par exemple. En outre, le seuil S1 choisi n'est pas constant, mais varie en fonction de la fréquence. A des fréquences choisies avec un pas de par exemple 0,2 MHz entre elles, on a associé un seuil déterminé empiriquement. Plus particulièrement, le seuil associé à chaque fréquence est choisi suffisamment bas pour conserver autant d'information utile que possible mais suffisamment haut pour éliminer autant que possible le bruit. Pour faire cela, on règle le seuil en observant la bande horizontale correspondant à la fréquence considérée dans l'image de la figure 17, pour que cette bande comporte autant de plages sombres que possible se détachant sur un fond clair.

La figure 17 représente l'image, fournie sur l'écran de l'appareil, de la projection des valeurs singulières dans le plan fréquence temporelle (ordonnées) - fréquence spatiale (abscisses). Pour une fréquence temporelle donnée, la fréquence spatiale indique la vitesse de propagation. En ce sens, le graphe de la figure 17 est du même genre que ceux des figures 6 et 7, où cependant la fréquence temporelle est en abscisses. A la figure 17, une échelle verticale à droite explicite les niveaux de gris comme expliqué en référence à la figure 6.

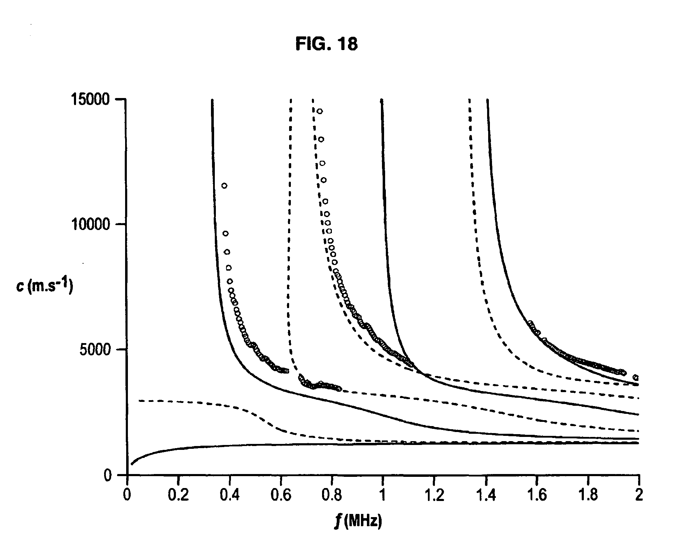

La figure 18 montre les vitesses de phase obtenues par les maximums de l'image de la figure 17. Les points obtenus sont figurés par des petits cercles. Les lignes en trait plein et en pointillés illustrent les trajectoires théoriques.

Les résultats obtenus pour certaines trajectoires sont assez satisfaisants, mais certaines trajectoires et parties de trajectoires ne sont pas obtenues. Les résultats sont moins complets qu'avec la plaque de cuivre. Ce n'est pas surprenant car l'os est un milieu plus bruité et atténuant que le cuivre, donc la qualité des signaux enregistrés est moindre.

## Revendications

1. Procédé ultrasonore de caractérisation d'un milieu, comprenant :

   - appliquer un signal d'excitation ultrasonore au milieu,
   - détecter une réponse constituée des signaux ultrasonores reçus en plusieurs sites de réception (12) situés à différentes distances d'un site d'émission (11) où le signal d'excitation a été appliqué,
   - à partir de l'ensemble des signaux ultrasonores reçus, extraire une cartographie des modes qui se sont propagés, dans un repère fréquence-vitesse de propagation,

   **caractérisé en ce que** pour chaque site de réception (12) la détection est réalisée successivement pour au moins deux signaux d'excitation différents, de façon que chaque site de réception voit passer au moins deux réponses dont les composantes vibratoires sont différentes et/ou différemment additionnées, **en ce qu'**on établit pour chaque fréquence une matrice $N^E$ x $N^R$ des niveaux d'énergie et des déphasages des réponses pour $N^R$ sites de réception et $N^E$ signaux d'émission différents et **en ce qu'**on soumet la matrice des réponses fréquentielles initiales à une décomposition en valeurs singulières dont il résulte pour chaque fréquence une base de vecteurs singuliers dans l'espace des excitations, et une base de vecteurs singuliers dans l'espace des ondes qui se sont propagées le long des récepteurs, ainsi que des valeurs singulières.

2.  Procédé selon la revendication 1, **caractérisé en ce que** les signaux d'excitation différents diffèrent par la distance entre leurs sites d'application respectifs et chaque site de réception.

3.  Procédé selon la revendication 2, **caractérisé en ce qu'**en dehors de la distance entre le site d'application et chaque site de réception les signaux d'excitation sont sensiblement identiques.

4.  Procédé selon l'une des revendications 1 à 3, **caractérisé en ce que** les signaux d'excitation différents sont appliqués à des instants différents, et on détecte séparément les réponses pour chaque signal d'excitation.

5.  Procédé selon l'une des revendications 1 à 4, **caractérisé en ce qu'**on détermine le spectre des composantes vibratoires de chaque signal ultrasonore reçu, en particulier par calcul de la transformée de Fourier temporelle de chaque signal reçu.

6.  Procédé selon l'une des revendications 1 à 5, **caractérisé en ce que** pour chaque fréquence on exprime la base des ondes planes dans la base des vecteurs singuliers de réception et on désigne celles des ondes planes qui participent le plus aux vecteurs singuliers de réception.

7.  Procédé selon l'une des revendications 1 à 5, **caractérisé en ce qu'**on utilise au moins deux émetteurs et on détermine pour chaque fréquence au moins une émission combinée des deux émetteurs, pour laquelle la réponse reçue aux sites de réception est optimisée en termes d'énergie reçue par rapport à l'énergie émise.

8.  Procédé selon l'une des revendications 1 à 5, **caractérisé en ce qu'**on utilise plusieurs émetteurs et on détermine pour chaque fréquence plusieurs émissions combinées des plusieurs émetteurs pour lesquelles les réponses fréquentielles respectives sont optimisées en termes d'énergie reçue par rapport à l'énergie émise.

9.  Procédé selon la revendication 8, **caractérisé en ce qu'**on sélectionne les réponses fréquentielles les plus énergétiques pour la détermination des ondes planes qui participent aux signaux de réception, et on élimine au moins une réponse fréquentielle peu énergétique.

10.  Procédé selon l'une des revendications 1 à 9, **caractérisé en ce qu'**on norme les réponses fréquentielles.

11.  Dispositif pour la mise en oeuvre d'un procédé selon l'une des revendications 1 à 10, comprenant une sonde (4) équipée de moyens d'émission ultrasonore (11) et d'une rangée de récepteurs ultrasonores (12), des moyens pour activer les moyens d'émission et recevoir les signaux provenant des récepteurs dans une fenêtre de temps après activation des moyens d'émission, et des moyens de traitement pour extraire des signaux reçus une cartographie des modes qui se sont propagés, dans un repère fréquence vitesse de propagation, **caractérisé en ce que** les moyens d'émission comprennent plusieurs émetteurs (11) et les moyens de traitement établissent la cartographie en combinant mathématiquement les signaux reçus en réponse à différents états d'activation des émetteurs, et **en ce que** les moyens de traitement réalisent :

- la transformée de Fourier temporelle de chaque signal reçu par chaque récepteur en réponse à chaque état d'activation, de façon à obtenir pour chaque fréquence une matrice $N^E$ X $N^R$ de $N^E$ réponses acquises par $N^R$ détecteurs en réponse à $N^E$ états d'activation,
- la décomposition en valeurs singulières de ces matrices pour tirer de chacune d'elles au moins un vecteur singulier formant partie constitutive d'une base de l'espace de réception,
- le calcul des coordonnées d'un vecteur représentant une onde plane donnée dans la base des vecteurs singuliers de réception.

12.  Dispositif selon la revendication 11, comprenant des moyens pour éliminer des vecteurs singuliers correspondant à des niveaux d'énergie relativement bas.

**Claims**

1. An ultrasonic method of characterizing a medium, comprising:

   - applying an ultrasonic excitation signal to the medium,
   - detecting a response constituted of the ultrasonic signals received at several receiving sites (12) situated at different distances from an emission site (11) where the excitation signal was applied,
   - from the set of ultrasonic signals received, extracting a map of the modes that have been propagated, in a frequency-propagation velocity reference system,

   **characterized in that** for each receiving site (12) the detection is carried out successively for at least two different excitation signals, so that each receiving site sees at least two responses whose vibratory components are different and/or are added differently, **in that** a matrix $N^E$ x $N^R$ of the energy levels and phase differences of the responses for $N^R$ different receiving sites and $N^E$ different emission signals is established for each frequency and **in that** the matrix of the initial frequency responses is subjected to singular value decomposition, the result of which for each frequency is a base of singular vectors in the space of the excitations, and a base of singular vectors in the space of the waves that were propagated along the receivers, as well as singular values.

2. The method according to claim 1, **characterized in that** the different excitation signals differ by the distance between their respective sites of application and each receiving site.

3. The method according to claim 2, **characterized in that** apart from the distance between the site of application and each receiving site, the excitation signals are substantially identical.

4. The method according to one of claims 1 to 3, **characterized in that** the different excitation signals are applied at different times, and the responses are detected separately for each excitation signal.

5. The method according to one of claims 1 to 4, **characterized in that** the spectrum of the vibratory components of each ultrasonic signal received is determined, in particular by calculating the temporal Fourier transform of each signal received.

6. The method according to one of claims 1 to 5, **characterized in that** for each frequency, the base of the plane waves is expressed in the base of the singular reception vectors and those of the plane waves that contribute most to the singular reception vectors are designated.

7. The method according to one of claims 1 to 5, **characterized in that** at least two emitters are used and for each frequency, at least one combined emission of the two emitters is determined, for which the response received at the receiving sites is optimized in terms of energy received with relation to the energy emitted.

8. The method according to one of claims 1 to 5, **characterized in that** several emitters are used and, for each frequency, several combined emissions of several emitters are determined, for which the respective frequency responses are optimized in terms of energy received with relation to the energy emitted.

9. The method according to claim 8, **characterized in that** the most energetic frequency responses are selected for determining the plane waves that contribute to the reception signals, and at least one low-energy frequency response is eliminated.

10. The method according to one of claims 1 to 9, **characterized in that** the frequency responses are normalized.

11. A device for implementing a method according to one of claims 1 to 10, comprising a probe (4) equipped with ultrasonic emission means (11) and a row of ultrasonic receivers (12), means for activating the emission means and receiving signals from the receivers in a time window after activation of the emission means, and processing means for extracting from the signals received a map of the modes that have been propagated, in a frequency-propagation velocity reference system, **characterized in that** the emission means comprise several emitters (11) and the processing means establish the map by mathematically combining the signals received in response to different activation states of the emitters, and **in that** the processing means carry out:

    - the temporal Fourier transform of each signal received by each receiver in response to each activation state, so as to obtain for each frequency a matrix $N^E$ x $N^R$ of $N^E$ responses acquired by $N^R$ detectors in response to $N^E$ activation states,

- singular value decomposition of these matrices for extracting from each of them at least one singular vector forming a component part of a base of the reception space,
- calculation of the coordinates of a vector representing a given plane wave in the base of the singular reception vectors.

**12.** The device according to claim 11, comprising means for eliminating singular vectors corresponding to relatively low energy levels.

**Patentansprüche**

**1.** Ultraschallverfahren zur Charakterisierung eines Mediums, umfassend:

- Anlegen eines Ultraschallanregungssignals an das Medium,
- Erfassen eines Antwortverhaltens, bestehend aus Ultraschallsignalen, die an verschiedenen Empfangsorten (12) empfangen werden, die sich in verschiedenen Abständen von einem Abstrahlungsort (11) befinden, an den das Anregungssignal angelegt worden ist,
- Extrahieren eines Mappings, nach Frequenz und Ausbreitungsgeschwindigkeit, der Moden, die sich ausgebreitet haben, aus der Gesamtheit der empfangenen Ultraschallsignale,

**dadurch gekennzeichnet, dass** für jeden Empfangsort (12) die Erfassung aufeinanderfolgend für mindestens zwei verschiedene Anregungssignale erfolgt, derart, dass jeder Empfangsort von mindestens zwei Antworten passiert wird, deren Schwingungskomponenten unterschiedlich sind und/oder sich auf unterschiedliche Weise summiert haben, dadurch, dass für jede Frequenz eine Matrix $N^E \times N^R$ der Energieterme und der Phasenänderungen der Antworten für $N^R$ Empfangsorte und $N^E$ verschiedene Sendesignale aufgestellt wird, und dadurch, dass die Matrix der anfänglichen Frequenzantworten einer Singulärwertzerlegung unterzogen wird, aus der für jede Frequenz eine Singulärvektorbasis im Raum der Anregungen und eine Singulärvektorbasis im Raum der Wellen, die sich entlang der Empfänger ausgebreitet haben, sowie Singulärwerte resultieren.

**2.** Verfahren nach Anspruch 1, **dadurch gekennzeichnet, dass** sich die verschiedenen Anregungssignale durch den Abstand zwischen ihren jeweiligen Applikationsorten und jedem Empfangsort unterscheiden.

**3.** Verfahren nach Anspruch 2, **dadurch gekennzeichnet, dass**, abgesehen vom Abstand zwischen dem Applikationsort und jedem Empfangsort, die Anregungssignale im Wesentlichen gleich sind.

**4.** Verfahren nach einem der Ansprüche 1 bis 3, **dadurch gekennzeichnet, dass** die verschiedenen Anregungssignale zu verschiedenen Zeitpunkten angelegt werden und für jedes Anregungssignal die Antworten separat erfasst werden.

**5.** Verfahren nach einem der Ansprüche 1 bis 4, **dadurch gekennzeichnet, dass** das Spektrum der Schwingungskomponenten jedes empfangenen Ultraschallsignals bestimmt wird und zwar insbesondere durch Berechnung der zeitlichen Fourier-Transformierten jedes empfangenen Signals.

**6.** Verfahren nach einem der Ansprüche 1 bis 5, **dadurch gekennzeichnet, dass** für jede Frequenz die Basis der ebenen Wellen in der Basis der Empfangssingulärvektoren ausgedrückt wird und jene ebenen Wellen bestimmt werden, die am meisten zu den Empfangssingulärvektoren beitragen.

**7.** Verfahren nach einem der Ansprüche 1 bis 5, **dadurch gekennzeichnet, dass** mindestens zwei Sender verwendet werden und für jede Frequenz mindestens eine kombinierte Abstrahlung von zwei Sendern bestimmt wird, für welche die an den Empfangsorten empfangene Antwort hinsichtlich der empfangenen Energie im Verhältnis zur ausgesendeten Energie optimiert wird.

**8.** Verfahren nach einem der Ansprüche 1 bis 5, **dadurch gekennzeichnet, dass** mehrere Sender verwendet werden und für jede Frequenz mehrere kombinierte Abstrahlungen von mehreren Sendern bestimmt werden, für welche die entsprechenden Frequenzantworten hinsichtlich der empfangenen Energie im Verhältnis zur ausgesendeten Energie optimiert werden.

**9.** Verfahren nach Anspruch 8, **dadurch gekennzeichnet, dass** für die Bestimmung der ebenen Wellen, die zum Empfangssignal beitragen, die Frequenzantworten mit den höchsten Energieanteilen ausgewählt werden und mindestens eine Frequenzantwort mit einem niedrigen Energieanteil eliminiert wird.

**10.** Verfahren nach einem der Ansprüche 1 bis 9, **dadurch gekennzeichnet, dass** die Frequenzantworten normiert werden.

**11.** Vorrichtung zur Durchführung eines Verfahrens nach einem der Ansprüche 1 bis 10, umfassend eine Sonde (4), die mit Mitteln (11) zum Aussenden von Ultraschall und einer Reihe von Ultraschallempfängern (12), Mitteln zum Aktivieren der Sendemittel und Entgegennehmen der Signale, die von den Empfängern kommen, in einem ersten Zeitfenster nach der Sendemittelaktivierung und Verarbeitungsmitteln zum Extrahieren eines Mappings, nach Frequenz und Ausbreitungsgeschwindigkeit, der Moden, die sich ausgebreitet haben, aus den empfangenen Signalen ausgestattet ist, **dadurch gekennzeichnet, dass** die Sendemittel mehrere Sender (11) umfassen und die Verarbeitungsmittel das Mapping erstellen, indem sie die als Antwort auf verschiedene Aktivierungszustände der Sender empfangenen Signale mathematisch kombinieren, und dadurch, dass die Verarbeitungsmittel Folgendes ausführen:

- die zeitliche Fourier-Transformation jedes Signals, das von jedem Empfänger als Antwort auf jeden Aktivierungszustand entgegengenommen wird, derart, dass für jede Frequenz eine Matrix $N^E$ x $N^R$ von $N^E$ Antworten erhalten wird, die von $N^R$ Gebern als Antwort auf $N^E$ Aktivierungszustände erfasst werden,
- die Singulärwertzerlegung dieser Matrizen, um aus jeder mindestens einen Singulärvektor zu erlangen, der einen Bestandteil einer Basis des Empfangsraums bildet,
- die Berechnung der Koordinaten eines Vektors, der eine bestimmte ebene Welle in der Basis der Empfangssingulärvektoren darstellt.

**12.** Vorrichtung nach Anspruch 11, umfassend Mittel zum Eliminieren von Singulärvektoren, die Termen relativ niedriger Energie entsprechen.

# FIG. 1

# FIG. 2

FIG. 3

FIG. 4

# FIG. 5

# FIG. 6

**FIG. 7**

**FIG. 8**

## FIG. 9

## FIG. 10

**FIG. 11**

**FIG. 12**

**FIG. 13**

SVD

**FIG. 14**

TF2D

**FIG. 15**

Th

## FIG. 16

## FIG. 17

## FIG. 18

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2839877
- WO 03045251 A1